# EUROPEAN PATENT SPECIFICATION

(11) **EP 3 795 086 B1**
(45) Date of publication and mention of the grant of the patent: **04.05.2022**
(21) Application number: 19306151.2
(22) Date of filing: 20.09.2019
(51) Int. Cl.: A61B 7/00

(54) **METHOD AND SYSTEM FOR MONITORING PHYSIOLOGICAL SIGNALS**
VERFAHREN UND SYSTEM ZUR ÜBERWACHUNG PHYSIOLOGISCHER SIGNALE
PROCÉDÉ ET SYSTÈME DE SURVEILLANCE DE SIGNAUX PHYSIOLOGIQUES

(43) Date of publication of application: 24.03.2021
(73) Proprietor: Mybrain Technologies, 92130 Issy-les-Moulineaux (FR)
(72) Inventor: NAVARRO-SUNE, Xavier, 75010 Paris (FR); POURCHIER, Nicolas, 13360 Roquevaire (FR); VAILHEN, Fabrice, 75010 Paris (FR); ATTAL, Yohan, 75010 Paris (FR)
(74) Representative: Icosa

(56) References cited:
- US-A1- 2018 014 741
- US-A1- 2019 239 772
- US-A1- 2019 247 010

## Description

### FIELD OF INVENTION

The present invention relates to the field of acoustic signal analysis. In particular, the present invention relates to the field of the analysis acoustic signal produced by a subject so as to monitor at least one breathing signal of said subject.

### BACKGROUND OF INVENTION

Nowadays innovations in wearable sensors have opened the possibility for monitoring human physiological functions out of the clinic environment. Early attempts in this direction employed actigraphy, which uses inertial sensors to measure activity of various body parts, with applications in sports and general health. More recent efforts have focused on recording cardiac activity, either electrically, via electrocardiograms (ECG), or optically, through photoplethysmograms (PPG). These require either a chest-strap or wrist-band, with the latter considered inconspicuous and comfortable to wear for most people, and the former quite uncomfortable.

The development of wearable devices for other modalities, such as respiration and neural activity, has been largely hampered by the inconvenience of their respective form factors - a chest-strap within respiration monitors is uncomfortable for long-term use, while the electroencephalogram (EEG) recorded from the scalp is cumbersome to set up, stigmatizing when out-of-the-clinic, and prone to artifacts.

Thanks to the recent advances, the health monitoring solutions starts to consider more than one single sensing modality. For example, US patent application 2018/014741 describes a wearable physiological monitoring device including plural EEG electrodes, an optical sensor comprised in an ear-plug structure configured to be mounted inside an ear of a user. This device is configured to measure the electroencephalographic signals using the EEG electrodes and the cardiac signal from the optical sensor.

Actual health monitoring solutions either not suitable for out-of-the-clinic measurement or uncomfortable for long-term use.

It is these drawbacks that the invention is intended more particularly to remedy by proposing an apparatus and a method for monitoring physiological signals of a subject from sounds produced by the subject.

US 2019/247010 discloses a biosensor system comprising:
- a microphone for detecting heartbeat sounds and respiration sounds from the ear canal;
- a gyroscope and an accelerometer to characterise the user's motion;
- means for determining a heart rate from the signal detected by the microphone;
- means for determining a respiration rate from the heart rate;

US 2019/239772 discloses a system comprising
- an earphone comprising: a microphone for detecting respiration sounds, a sensor configured to measure a heartbeat signal and at least one of an accelerometer or a gyroscope;
- a processor receiving an input signal from the microphone to determine a respiration rate based on the detected respiration sounds;
- a processor receiving an input signal from the accelerometer or gyroscope to determine a respiration rate based on this input
- a processor receiving an input signal from the heartbeat sensor and to determine a respiration rate based on peaks of the heartbeat signals.

### SUMMARY

The present invention relates to a method for providing an estimation of physiological signals of a subject from sounds produced by the subject according to claim 1.

The method may comprise monitoring physiological signals of a subject from sounds produced by the subject, said method comprising the following steps:
- receiving recorded sounds comprising sounds originating from the chest of the subject and being transmitted by biological tissues of the subject to the ears of the subject, wherein said recorded sounds are recorded by at least one sound recording element positioned inside at least one earcup of headphones worn by the subject;
- receiving signals from an accelerometer and a gyroscope being recorded simultaneously with the recorded sounds;
- removing noise originating from sounds propagating in the environment external to the earcups from the recorded sounds to obtain denoised sounds;
- extracting from the denoised sounds cardiac peaks, corresponding to systolic and diastolic sounds, and residual sounds comprising information generated by the breathing signal;
- detecting heart beats from the cardiac peaks sounds and calculating inter-beat intervals from the heart beats;
- extracting a first estimation of the breathing signal from the inter-beat intervals presenting respiratory sinus arrhythmia;
- extracting a second estimation of the breathing signal from residual sounds;
- extracting a third estimation of the breathing signal and motion artifacts from the signals of the accelerometer and the gyroscope;
- calculating the breathing signal by combining the first, the second and the third estimation of the breathing signal.

The present invention advantageously allows to obtain from sound recorded in proximity of the ear canal and the inertial motion signals a robust estimation of the cardiac and respiratory signal. If one side the cardiac signal is relatively intense to be easily detected from the recorded sounds, the evaluation of respiratory signals is particularly challenging due to their low intensity. The present method thanks to the fusion of three different estimations of the breathing signal is advantageously able to provide a reliable measure of the breathing signal.

According to one embodiment, the extraction of the cardiac peaks sounds comprises a step of enhancing the peaks in the recorded sounds and a step of detecting the cardiac peaks sounds using a discrete wavelet transform.

According to one embodiment, the step of extracting the first estimation of the breathing signal comprises the application of Fast Fourier Transform to the resampled inter-beat intervals and the selection of the low frequency component.

According to one embodiment, the step of extracting the second estimation of the breathing signal from the residual sound is performed using time-frequency analysis and periodicity detection.

According to one embodiment, the step of extracting the third estimation of the breathing signal comprises the use of principal component analysis decomposition, fast Fourier spectral computation and component detection of the signals of the accelerometer and the gyroscope.

According to one embodiment, wherein a fusion algorithm is used to combine the first, the second and the third estimation of the breathing signal.

According to one embodiment, the method further comprises the step of receiving electroencephalographic signals of the subject recorded simultaneously to the recorded sounds.

The present invention further relates to a computer program product for monitoring physiological signals of a subject, the computer program product comprising instructions which, when the program is executed by a computer, cause the computer to carry out the steps of the method according to any one of embodiments here above.

Yet the present invention relates to a computer-readable storage medium comprising instructions which, when the program is executed by a computer, cause the computer to carry out the steps of the method according to any one of embodiments here above.

The present invention also relates to a system for the monitoring of physiological signals of a subject from recorded sounds comprising:
- an acquisition module configured to record sounds originating from the chest of the subject and being transmitted by biological tissues of the subject to the ears of the subject, wherein the recorded sounds are recorded by at least one sound recording element positioned inside at least one earcup of headphones worn by the subject; and to record, simultaneously with the recorded sounds, signals from an accelerometer and a gyroscope;
- a denoising module configured to remove noise originating from sounds propagating in the environment external to the earcups from the recorded sounds to obtain denoised sounds;
- an extraction module configured to extract from the denoised sounds cardiac peaks, corresponding to systolic and diastolic sounds, and residual sounds comprising information generated by the breathing signal;
- a cardiac analysis module configured to detect the heart beats from the cardiac peaks sounds and calculating inter-beat intervals from the heart beats;
- a respiratory analysis module configured to extract from the denoised sounds a first estimation of the breathing signal from the inter-beat intervals, extract a second estimation of the breathing signal from residual sounds, extract a third estimation of the breathing signal and motion artifacts from the signal of the accelerometer and the gyroscope, and calculate the breathing signal combining the first, the second and the third estimation of the breathing signal.

According to one embodiment, the system further comprises headphones comprising two earcups configured to amplify the sound originating from the chest of the subject and being transmitted by biological tissues of the subject to the ears of the subject.

According to one embodiment, the at least one sound recording element is positioned inside at least one of the earcups.

According to one embodiment, the system further comprises an external sound recording element positioned on the outside of at least one of the earcups so as to record the sounds propagating in the environment external to the earcups.

According to one embodiment, the earcups of the headphones are circumaural headphones or supra-aural headphones.

According to one embodiment, the denoising module is further configured to perform noise cancellation using an adaptative filter to remove the recorded sounds propagating in the environment external to the earcups from the recorded sounds

According to one embodiment, the system comprises at least two electrodes configured to acquire the electroencephalographic signal of the subject. In one embodiment, the at least two electrodes are textile electrodes comprised in the earcups so that the textile electrodes rest against the skin disposed over the mastoid processes when the headphones is worn by a subject, the use of textile electrodes ensure comfort to the subject.

### DEFINITIONS

In the present invention, the following terms have the following meanings:
- **"Subject"** refers to a mammal, preferably a human. In the sense of the present invention, a subject may be an individual having any mental or physical disorder requiring regular or frequent medication or may be a patient, i.e. a person receiving medical attention, undergoing or having underwent a medical treatment, or monitored for the development of a disease.
- **"Headphones"** refer to a pair of small loudspeaker drivers worn on or around the head over a user's ears.
- **"Electroencephalogram"** refers to the record of the electrical activity of the brain of a subject.

### BRIEF DESCRIPTION OF THE DRAWINGS

**Figure 1** is a work flow providing a schematic representation of the steps of the method according to one embodiment of the present invention.
**Figure 2** is a work flow providing a schematic representation of the steps performed for the extraction from the denoised sounds cardiac peaks and the residual sounds.
**Figure 3** is a work flow providing a schematic representation of the steps performed for the extraction of a first estimation of the breathing signal from inter-beat intervals.
**Figure 4** is a work flow providing a schematic representation of the steps performed for the extraction of a second estimation of the breathing signal from residual sounds.
**Figure 5** is a work flow providing a schematic representation of the steps performed for the extraction of a third estimation of the breathing signal and motion artifacts from the signals of the accelerometer and the gyroscope.
**Figure 6** is a work flow providing a schematic representation of the steps performed for calculation of the breathing signal by combining the first, the second and the third estimation of the breathing signal.
**Figure 7** is a schematic representation of the system for the monitoring of physiological signals of a subject from recorded sounds according to one embodiment of the present invention.
**Figure 8** is a schematic representation of the system for the monitoring of physiological signals of a subject from recorded sounds comprising the headphones.

### DETAILED DESCRIPTION

The following detailed description will be better understood when read in conjunction with the drawings. For the purpose of illustrating, the system is shown in the preferred embodiments and the block diagrams, comprising the steps of the method, are shown in the preferred embodiments. It should be understood, however that the application is not limited to the precise arrangements, structures, features, embodiments, and aspect shown. The drawings are not drawn to scale and are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

The present invention relates to a method 100 for monitoring physiological signals of a subject from the acoustic analysis of sounds produced by physiological process in a subject. Among the multiple physiological process taking place in the human body, heart beating and respiration are among those that produce the most perceptible sounds.

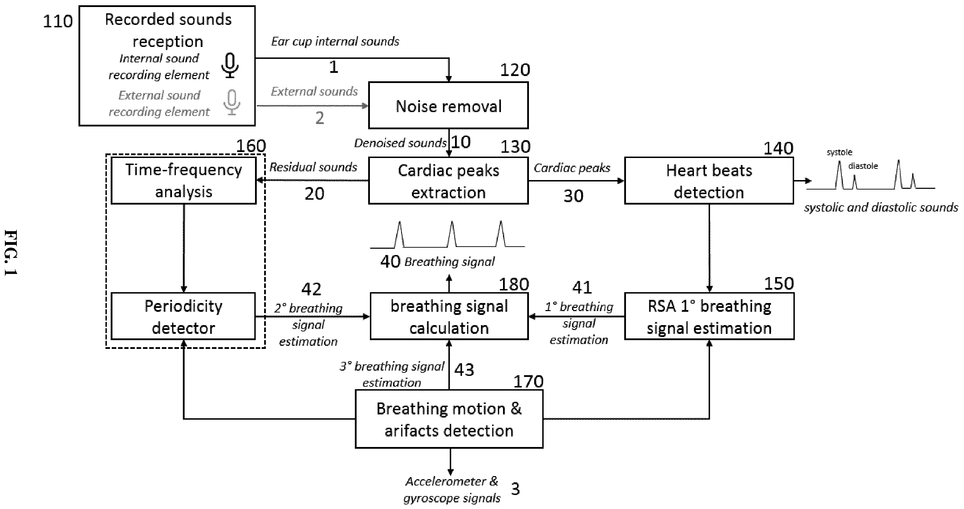

According to the embodiment illustrated in Figure 1, an initial step of the method consists in the reception 110 of recorded sounds 1 comprising sounds originating from the chest of the subject and being transmitted by biological tissues of the subject to the ears of the subject. Said sounds originating from the chest are produced at least in part from the heart beating and respiration.

In one embodiment, the recorded sounds 1 are recorded by at least one sound recording element positioned inside at least one earcup of headphones worn by the subject, the sound recording element being notably a microphone. The earcup of the headphone generally present a curved body where the convex surface faces the ear of the subject sealing at least partially the volume between the ear and the earcup from the external environment while the concave surface faces the external environment. In one embodiment, the at least one sound recording element is disposed on the convex surface of the earcup. However, even if the shape of the earcups is configured to seal the concave surface of the earcup and the sound recording element from the external environment, the acoustic isolation is not complete, and therefore the sound recorded from the sound recording element comprises also a component originating from the sounds propagating in the environment external to the earcups 2.

According to one embodiment, the initial step 110 of the method further comprises the reception of sounds propagating in the environment external to the earcups 2. Said sounds propagating in the environment external to the earcups 2 may be recorded by using an external sound recording element positioned on the outside of at least one of the earcups of the headphones.

In one embodiment, the earcups of the headphones are circumaural headphones or supra-aural headphones. In circumaural headphones have circular or ellipsoid earcup that encompass the ears. Because these headphones completely surround the ear, circumaural headphones can be designed to fully seal against the head to attenuate external noise. Supra-aural headphones have pads that press against the ears, rather than around them. This type of headphone generally tends to be smaller and lighter than circumaural headphones, resulting in less attenuation of outside noise.

In one embodiment, the method further receives as input signals from an accelerometer and a gyroscope being recorded simultaneously with the recorded sounds. The signals may be recorded directly from an accelerometer and a gyroscope integrated into the headphones.

According to one embodiment, the method comprises a step of removal of noise 120 originating from sounds propagating in the environment external to the earcups from the recorded sounds 1 to obtain denoised sounds 10. Standard noise cancellation technique, known by the person skilled in the art.

According to the embodiment wherein the method comprises the reception of sounds propagating in the environment external to the earcups, the step of removal of noise uses an active noise cancellation algorithm using for example adaptative filtering and the sounds propagating in the environment external to the earcups.

In one embodiment, the method comprises a step of extracting 130 from the denoised sounds 10 cardiac peaks sounds 30, corresponding to systolic and diastolic sounds, and residual sounds 20 comprising information generated by the breathing signal.

According to one embodiment, the extraction of the cardiac peaks sounds comprises a step of enhancing the peaks in the recorded sounds 1 and a step of detecting the cardiac peaks sounds using a discrete wavelet transform.

Cardiac sounds are noticeable as periodic double peak, corresponding to systolic and diastolic sounds. In one embodiment, the discrete wavelet transform (DWT) is used in this step to enhance and detect the period cardiac peaks sounds 30. Because discrete wavelet transform localizes patterns in signals to different scales, relevant signal features can be preserved while removing noise. In one embodiment, a wavelet denoising based on 4^{th} order symlets is used. This pattern is matched into the original sound and extracted to obtain two signals: one related to cardiac sounds and another containing noise and respiratory sounds.

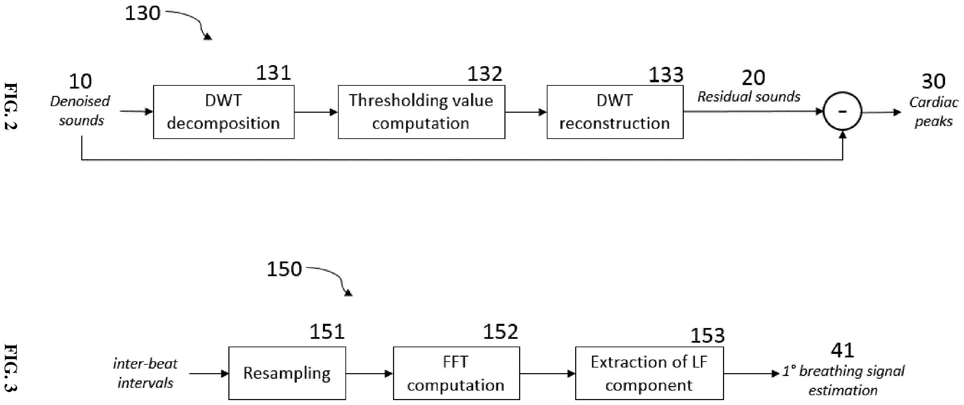

In one example illustrated in Figure 2, the denoised sounds 10 is decomposed 131 using the wavelet of 4^{th} order symlets. Following the decomposition of the denoised sounds 10, a thresholding value is computed 132 in order to isolate the cardiac peaks sounds from other components in the sounds using a method know by the person skilled in the art such as Birgé-Massart strategy. The following step consists in the reconstruction by discrete wavelet transform 133 of the residual sounds 20, meaning sounds not comprising cardiac peaks. Finally, the cardiac peaks sounds 30 are obtained by subtraction of the residual sounds 20 from the denoised sounds 10.

According to one embodiment, the method comprises the step of detecting heart beats 140 from the cardiac peaks sounds 30.

In one example, the step 140 comprises the application of a band-pass filter to the cardiac peaks sounds 30 and the application of a derivative operation to the filtered sounds. In this example, the derivative operation is followed by a squared operation and a moving window integration of the resulting sounds. Finally, applying an adaptative threshold to the heart beats are detected.

According to one embodiment, the method further comprises the calculation of the inter-beat intervals from the heart beats. In one alternative embodiment, the inter-beat intervals are calculated from the cardiac peak sounds 30.

According to one embodiment, the method of the present invention comprises a step 150 of extracting a first estimation of the breathing signal 41 from the inter-beat intervals presenting respiratory sinus arrhythmia (RSA). Indeed, respiratory sinus arrhythmia (RSA) is a prominent component of heart rate variability, this is the phenomenon by which the R-R interval on a cardiac signal is shortened during inspiration and prolonged during expiration.

According to one embodiment illustrated in Figure 3, the method further comprises the step of resampling the inter-beat intervals 151 and computing a fast Fourier transform of the resampled inter-beat intervals 152. In one embodiment, the first estimation of the breathing signal 41 is extracted from the selection of the low frequency components of the fast Fourier transform 153.

According to an alternative embodiment, the QRS complexes are detected using a waveform decomposition strategy. An exemplary decomposition algorithm may use a 2-poles 2-zeros resonator filter centered at 17 Hz with a bandwidth of 6 Hz to highlight the sharp edges of the QRS and smooth out the other ECG waveforms. Once filtered, a simple adaptive threshold filter may be used to select the QRS fiducial point used by the second stage of the respiration detection algorithm. After the QRS annotation, the heart rate variability may be calculated over the duration of the sampled ECG data, allocating an x-value equal to the midpoint between the R peaks. This heart rate variability is then inverted and resampled at 1000 Hz. The heart rate variability is considered to be the respiration waveform, where the local minima are considered the point of maximum expiration due to the decreased heart rate, and the local maxima are the points of maximum inspiration for each breath.

According to one embodiment, the method comprises a step 160 of extracting a second estimation of the breathing signal 42 from residual sounds 20.

According to one embodiment, the step 160 of extracting the second estimation of the breathing signal 42 from the residual sound is performed using time-frequency analysis and periodicity detection.

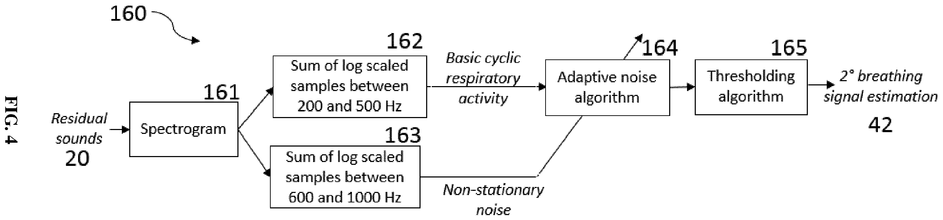

In one example illustrate in Figure 4, the step 160 comprises a first step of obtaining a spectrogram 161 of the residual sounds 20. Two frequency regions are selected on the spectrogram: one region corresponding to the basic cyclic respiratory activity (200 - 500 Hz) and one region corresponding to non-stationary noise manifestations with broadband bursts in the context of the total observation time (600 - 1000 Hz). In this example, the integral power synthesis signal is performed in the selected frequency range 200 - 500 Hz as sum of log scaled samples of power spectral density 162 obtaining a synthesized signal of basic cyclic respiratory activity. For the region comprising the frequencies 600 - 1000 Hz, the integral power synthesis signal is performed in the selected frequency range as sum of log scaled samples of power spectral density 163 obtaining a synthesized signal of non-stationary noise. Synthesized signal of basic cyclic respiratory activity is used as reference for detection of respiratory cycles. However, the signal component may contain noise spikes, which considerably complicates detection of respiratory cycles even after the first denoising step. Indeed, in most cases, recording of respiratory sounds is associated with the imposition of secondary and non-informative components as physiological (secondary noise, wheezing) and non-physiological nature interference. Assuming that the synthesized signal of basic cyclic respiratory activity is some kind additive mixture of the actual main component of respiratory activity and component of the non-stationary noise, an adaptive noise filtering algorithm 164 using the basic cyclic respiratory activity on the second frequency band is used for the cancelation of noise. Through this approach the respiratory activity signal was obtained as filtering result, given that the desired signal respiratory activity and respiratory noise signal power bursts are not correlated with each other. In this example, the output signal of the adaptive filter is additional filtered with a cutoff frequency around 4 Hz, which eliminates the influence of high frequency components of the breathing signal. A threshold algorithm 165 is further used for the extraction of the second estimation of the breathing signal 42.

According to one embodiment, the method of the present invention comprises a step 170 of extracting a third estimation of the breathing signal 43 and motion artifacts from the signals of the accelerometer and the gyroscope 3.

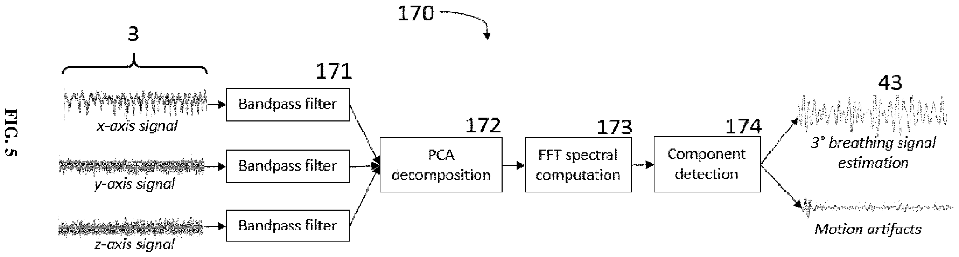

According to one embodiment illustrated in Figure 5, the step 170 comprises the application of a bandpass filter to the signals of the accelerometer and/or the gyroscope 3, the bandpass filter 171 being applied separately to the signal recorded from the 3 axis of the accelerometer and/or the 3 axis of the gyroscope. In one embodiment, principal component analysis decomposition 172 is used with the filtered signals of the accelerometer and/or the gyroscope and for the resulting signals the fast Fourier transform spectral 173 is computed obtaining signals in the frequency domain. In one embodiment, the third estimation of the breathing signal 43 and motion artifacts are extracted from the signals in the frequency domain using component detection 174.

According to one embodiment, the method comprises a step of calculating 180 the breathing signal 40 by combining the first 41, the second 42 and the third estimation of the breathing signal 43.

According to one embodiment, a fusion algorithm is used to combine the first 41, the second 42 and the third estimation 43 of the breathing signal. The combination of estimations can be done by several ways known by the man skilled in the art, such as using an ensemble learning, in particular cascading, which concatenates individual classifiers to obtain a more accurate result.

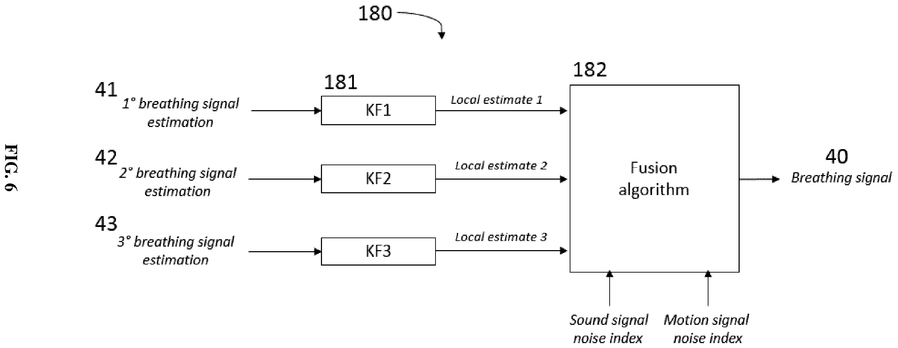

According to one example illustrated in Figure 6, the fusion algorithm is configured to adapt to different acquisition scenarios: depending on environmental noise and movement patterns, one of the three breathing signal estimation is considered more effective and therefore its weight increased with respect the others. For each breathing signal estimation (41, 42, 43) Kalman filtering (KF) 181 is used to obtain respective local estimates. Thus, two signal quality indices evaluating the quality of the signals are computed, notably a sound signal noise index using the external sounds 2 and/or the residual sounds 20 and a motion signal noise index using the accelerometer and/or gyroscope signals 3. The two signal quality indices are used to weight each local estimate based on previous reference data (i.e. learning dataset). Local estimates from individual Kalman-filters need to be fused in a manner that takes into account the uncertainty associated with each estimate (signal quality indices) and previous information from a learning dataset. State vector fusion methods use a bank of Kalman filters to obtain local estimates which are then fused to obtain an improved breathing signal 40 which is an accurate robust estimation of the breathing signal using signal quality indices and a modified Kalman Filter fusion framework 182 which uses the signal quality indices to adaptively update the Kalman Filter noise covariance estimate. The signal quality indices are derived in real time and therefore no assumptions concerning the signal-to-noise ratio are required. The main advantage of this approach is the inclusion of a signal quality metric (as well as the past behavior of each signal) to control the Kalman Filter noise covariance estimate and decide automatically how to weight each source of information.

According to one embodiment, the method further comprises the step of receiving electroencephalographic signals of the subject recorded simultaneously to the recorded sounds from which are estimated the physiological signals.

According to one embodiment, electroencephalographic signals is recorded synchronized with ECG and respiration so time-locked analysis can be performed in real time. In this embodiment, it is possible for instance to correlate event related potentials using heartbeats or inspiratory marks.

The present invention further relates to a computer program product for monitoring physiological signals of a subject, the computer program product comprising instructions which, when the program is executed by a computer, cause the computer to carry out the steps of the method according to any one of the embodiments described hereabove.

The computer program product to perform the method as described above may be written as computer programs, code segments, instructions or any combination thereof, for individually or collectively instructing or configuring the processor or computer to operate as a machine or special-purpose computer to perform the operations performed by hardware components. In one example, the computer program product includes machine code that is directly executed by a processor or a computer, such as machine code produced by a compiler. In another example, the computer program product includes higher-level code that is executed by a processor or a computer using an interpreter. Programmers of ordinary skill in the art can readily write the instructions or software based on the block diagrams and the flow charts illustrated in the drawings and the corresponding descriptions in the specification, which disclose algorithms for performing the operations of the method as described above.

The present invention further relates to a non-transitory computer-readable storage medium comprising instructions which, when the computer program is executed by a data processing system, cause the data processing system to carry out the steps of the method according to any one of the embodiments described hereabove.

Computer programs implementing the method of the present embodiments can commonly be distributed to users on a distribution computer-readable storage medium such as, but not limited to, an SD card, an external storage device, a microchip, a flash memory device, a portable hard drive and software websites. From the distribution medium, the computer programs can be copied to a hard disk or a similar intermediate storage medium. The computer programs can be run by loading the computer instructions either from their distribution medium or their intermediate storage medium into the execution memory of the computer, configuring the computer to act in accordance with the method of this invention. All these operations are well- known to those skilled in the art of computer systems.

The instructions or software to control a processor or computer to implement the hardware components and perform the methods as described above, and any associated data, data files, and data structures, are recorded, stored, or fixed in or on one or more non-transitory computer-readable storage media. Examples of a non-transitory computer-readable storage medium include read-only memory (ROM), random-access memory (RAM), flash memory, CD- ROMs, CD- Rs, CD+ Rs, CD- RWs, CD+ RWs, DVD- ROMs, DVD- Rs, DVD+ Rs, DVD- RWs, DVD+ RWs, DVD- RAMs, BD- ROMs, BD- Rs, BD- R LTHs, BD- REs, magnetic tapes, floppy disks, magneto-optical data storage devices, optical data storage devices, hard disks, solid-state disks, and any device known to one of ordinary skill in the art that is capable of storing the instructions or software and any associated data, data files, and data structures in a non-transitory manner and providing the instructions or software and any associated data, data files, and data structures to a processor or computer so that the processor or computer can execute the instructions. In one example, the instructions or software and any associated data, data files, and data structures are distributed over network-coupled computer systems so that the instructions and software and any associated data, data files, and data structures are stored, accessed, and executed in a distributed fashion by the processor or computer.

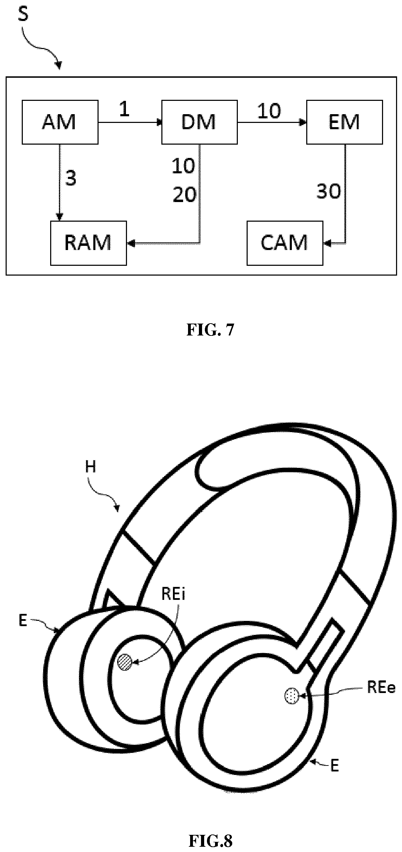

Yet another aspect of the present invention concerns a system for the monitoring of physiological signals of a subject from recorded sounds. Said system S comprises a plurality of modules cooperating with each other's as shown in Figure 7.

According to one embodiment, the system comprises an acquisition module AM configured to receive recorded sounds originating from the chest of the subject and being transmitted by biological tissues of the subject to the ears of the subject. Said recorded sounds 1 are recorded by means of at least one sound recording element positioned inside at least one earcup of headphones worn by the subject. In this embodiment, the acquisition module AM is further configured to receive, simultaneously with the recorded sounds, signals recorded from an accelerometer and a gyroscope 3.

Alternatively, the acquisition module AM is configured to record sounds originating from the chest of the subject and being transmitted by biological tissues of the subject to the ears of the subject using at least one sound recording element positioned inside at least one earcup of headphones worn by the subject. The acquisition module AM may be further configured to record, simultaneously with the recorded sounds, signals from an accelerometer and a gyroscope 3.

As shown in Figure 8, according to one embodiment, the system comprises headphones H comprising two earcups E. According to one embodiment, the earcups E of the headphones H are circumaural headphones or supra-aural headphones.

In one embodiment, the earcups E are configured to amplify the sound originating from the chest of the subject and being transmitted by biological tissues of the subject to the ears of the subject. In this embodiment, the earcups E have a curved body where the convex surface faces the ear of the subject sealing at least partially the volume between the ear and the earcup from the external environment while the concave surface faces the external environment. The convex surface facing the ear of the subject is configured to be a resonant cavity capable of attenuating some frequencies, such as the frequencies associated to of the noise in the external environment, and/or to amplify some other frequencies, such as the frequencies associated to the breathing sound produced by the breathing activity of the subject.

According to one embodiment, the at least one sound recording element RE is positioned inside one of the two earcups E of the headphones H.

According to one embodiment, the system comprises a denoising module DM configured to remove noise originating from sounds propagating in the environment external to the earcups from the recorded sounds 1 to obtain denoised sounds 10. Standard noise cancellation technique, known by the person skilled in the art, may be implemented in this module.

According to one embodiment, the system further comprises an external sound recording element RE positioned on the outside of at least one of the earcups so as to record the sounds propagating in the environment external to the earcups 2. According to this embodiment, the denoising module DM is configured to implement an active noise cancellation algorithm using for example adaptative filtering and the sounds propagating in the environment external to the earcups 2.

According to one embodiment, the system comprises an extraction module EM configured to extract from the denoised sounds 10 cardiac peaks 30, corresponding to systolic and diastolic sounds, and residual sounds 20 comprising information generated by the breathing signal.

According to one embodiment, the extraction module EM is configured to enhance the peaks in the recorded sounds 1 and to detect the cardiac peaks sounds using a discrete wavelet transform. In one embodiment, the extraction module EM is configured to use the discrete wavelet transform (DWT) to enhance and detect the period cardiac peaks sounds 30. Because discrete wavelet transform localizes patterns in signals to different scales, relevant signal features can be preserved while removing noise. In one embodiment, a wavelet denoising based on 4^{th} order symlets is used. This pattern is matched into the original sound and extracted to obtain two signals: one related to cardiac sounds and another containing noise and respiratory sounds.

In one example, the extraction module EM is configured to decompose the denoised sounds 10 using the wavelet of 4^{th} order symlets, compute a thresholding value in order to isolate the cardiac peaks sounds from others components in the sounds using known method, such as Birgé-Massart strategy, and to reconstruct by discrete wavelet transform the residual sounds 20, being sounds not comprising cardiac peaks.

Finally, the extraction module EM is configured to obtain the cardiac peaks sounds 30 are by the subtraction of the residual sounds 20 from the denoised sounds 10.

According to one embodiment, the system comprises a cardiac analysis module CAM configured to detect the heart beats from the cardiac peaks sounds 30 and calculating inter-beat intervals from the heart beats.

In one embodiment, the cardiac analysis module CAM is configured to band-pass filter the cardiac peaks sounds 30 and to implement the following operation on the filtered signals to detect the heart beats: a derivative operation, a squared operation, a moving window integration and an adaptative threshold. The cardiac analysis module CAM is further configured to calculation of the inter-beat intervals from the heart beats.

According to one embodiment, the system further comprises a respiratory analysis module RAM configured to extract from the denoised sounds 10 a first estimation of the breathing signal from the inter-beat intervals, to extract a second estimation of the breathing signal from residual sounds 20, to extract a third estimation of the breathing signal and motion artifacts from the signal of the accelerometer and the gyroscope, and to calculate the breathing signal combining the first, the second and the third estimation of the breathing signal

According to one embodiment, the respiratory analysis module RAM is configured to resample the inter-beat intervals, compute a fast Fourier transform of the resampled inter-beat intervals and extract the first estimation of the breathing signal from the low frequency components of the fast Fourier transform.

According to one embodiment, the respiratory analysis module RAM is configured to extract the second estimation of the breathing signal from the residual sound 20 using time-frequency analysis and periodicity detection.

According to one example, the respiratory analysis module RAM is configured to perform a first step to obtain a spectrogram of the residual sounds 20. Two frequency regions are selected on the spectrogram: one region corresponding to the basic cyclic respiratory activity (200 - 500 Hz) and one region corresponding to non-stationary noise manifestations with broadband bursts in the context of the total observation time (600 - 1000 Hz). In this example, the respiratory analysis module RAM further performs the integral power synthesis signal in the selected frequency range 200 - 500 Hz as sum of log scaled samples of power spectral density obtaining a synthesized signal of basic cyclic respiratory activity. For the region comprising the frequencies 600 - 1000 Hz, the integral power synthesis signal is performed by the respiratory analysis module RAM in the selected frequency range as sum of log scaled samples of power spectral density obtaining a synthesized signal of non-stationary noise. The synthesized signal of basic cyclic respiratory activity is used as reference for detection of respiratory cycles. An adaptive noise filtering algorithm using the basic cyclic respiratory activity on the second frequency band is used for the cancelation of noise by the respiratory analysis module RAM. This approach allows to obtain the respiratory activity signal as filtering result, given that the desired signal respiratory activity and respiratory noise signal power bursts are not correlated with each other. In this example, the respiratory analysis module RAM is further configured to filtered with a cutoff frequency around 4 Hz the output signal of the adaptive filter in order to eliminate the influence of high frequency components of the breathing signal. A threshold algorithm is further used for the extraction of the second estimation of the breathing signal 42.

According to one embodiment, the respiratory analysis module RAM is configured to extract a third estimation of the breathing signal and motion artifacts from the signal of the accelerometer and the gyroscope 3.

In one embodiment, the respiratory analysis module RAM is configured to bandpass filter separately the signals of the accelerometer and/or the gyroscope 3 recorded from the three axis of the accelerometer and/or the three axis of the gyroscope. In one embodiment, the respiratory analysis module RAM is configured to perform principal component analysis decomposition with the filtered signals of the accelerometer and/or the gyroscope and compute fast Fourier spectral transform to obtain signals in the frequency domain. According to one embodiment, the third estimation of the breathing signal and motion artifacts are extracted by the respiratory analysis module RAM from the signals in the frequency domain using component detection.

According to one embodiment, the method comprises a step of calculating the breathing signal by combining the first, the second and the third estimation of the breathing signal.

According to one embodiment, the respiratory analysis module RAM is configured to use a fusion of classifications to combine the first, the second and the third estimation of the breathing signal.

While various embodiments have been described and illustrated, the detailed description is not to be construed as being limited hereto. Various modifications can be made to the embodiments by those skilled in the art without departing from the true spirit and scope of the disclosure as defined by the claims.

## Claims

1. A computer-implemented method for providing an estimation of breathing signals of a subject, said method comprising:
- receiving recorded sounds (110) comprising sounds originating from a chest of a subject and being transmitted by biological tissues of the subject to the ears of the subject, wherein said recorded sounds (1) are previously recorded by at least one sound recording element positioned inside at least one earcup of headphones worn by the subject;
- receiving signals from an accelerometer and a gyroscope (3) which have been recorded simultaneously with the recorded sounds;
- extracting from the recorded sounds cardiac peaks (130), corresponding to systolic and diastolic sounds, and residual sounds (20) comprising information generated by respiration of the subject;
- detecting heart beats from the cardiac peaks (30) and calculating inter-beat intervals from the heart beats;
- extracting a first estimation of a breathing signal (41) from the inter-beat intervals (150) presenting respiratory sinus arrhythmia (RSA);
- extracting a second estimation of the breathing signal (42) from residual sounds (160);
- extracting a third estimation of the breathing signal (43) and motion artifacts from the signals of the accelerometer and the gyroscope (170);
- calculating (180) a fourth estimation of the breathing signal (40) by combining the first (41), the second (42) and the third (43) estimation of the breathing signal, and
- providing the fourth estimation of the breathing signal (40) for health monitoring.

2. The method according to claim **1,** wherein extracting of the cardiac peaks sounds (130) comprises a step of enhancing the peaks in the recorded sounds and a step of detecting the cardiac peaks sounds using a discrete wavelet transform.

3. The method according to either one of claims **1** or **2**, wherein the step of extracting the first estimation of the breathing signal (150) comprises the application of Fast Fourier Transform to the resampled inter-beat intervals and the selection of the low frequency component.

4. The method according to any one of claims **1** to 3, wherein the step of extracting the second estimation of the breathing signal (160) from the residual sounds (20) is performed using time-frequency analysis and periodicity detection.

5. The method according to any one of claims **1** to 4, wherein the step of extracting the third estimation of the breathing signal (170) comprises the use of principal component analysis decomposition, fast Fourier spectral computation and component detection of the signals of the accelerometer and the gyroscope.

6. The method according to any one of claims **1** to 5, wherein a fusion algorithm (182) is used to combine the first (41), the second (42) and the third estimation of the breathing signal (43).

7. The method according to any one of claims **1** to 6, further comprising the step of receiving electroencephalographic signals of the subject recorded simultaneously to the recorded sounds.

8. A computer program product for monitoring physiological signals of a subject, the computer program product comprising instructions which, when the program is executed by a computer, cause the computer to carry out the steps of the method according to any one of claims **1** to 7.

9. A system (S) for providing an estimation of breathing signals of a subject from recorded sounds comprising:
- an input module (AM) configured to receive:
• recorded sounds (1) acquired using at least one sound recording element (REi) positioned inside at least one earcup of headphones worn by the subject, said recorded sound (1) originating from a chest of the subject and being transmitted by biological tissues of the subject to the ears of the subject;
• signals from an accelerometer and a gyroscope (3), said signals which have been acquired simultaneously with the recorded sounds;
- an extraction module (EM) configured to extract from the recorded sounds (1) cardiac peaks (30), corresponding to systolic and diastolic sounds, and residual sounds (20) comprising information generated by respiration of the subject;
- a cardiac analysis module (CAM) configured to detect the heart beats from the cardiac peaks sounds (30) and calculating inter-beat intervals from the heart beats;
- a respiratory analysis module (RAM) configured to extract from the recorded sounds (10) a first estimation of a breathing signal from the inter-beat intervals, extract a second estimation of the breathing signal from residual sounds (20), extract a third estimation of the breathing signal and motion artifacts from the signals of the accelerometer and the gyroscope, and calculate a fourth estimation of the breathing signal combining the first, the second and the third estimation of the breathing signal.

10. The system according to claim 9, further comprising headphones (H) comprising two earcups (E) configured to amplify the sound originating from the chest of the subject and being transmitted by biological tissues of the subject to the ears of the subject.

11. The system according to claim 10, , wherein the at least one sound recording element (REi) is positioned inside at least one of the earcups (E).

12. The system according to either one of claims 10 or 11, further comprising an external sound recording element (REe) positioned on the outside of at least one of the earcups (E) so as to record sounds propagating in an environment external to the at least one earcups (2).

13. The system according to any one of claims 10 to 12, wherein the earcups (E) of the headphones (H) are circumaural headphones or supra-aural headphones.

14. The system according to either one of claim 12 13, further comprising a denoising module (DM) configured to receive the sounds propagating in the environment external to the earcups (2) and remove from the recorded sounds (1) a part of a noise using said sounds propagating in the environment external to the earcups (2).

## Patentansprüche

1. Computerimplementiertes Verfahren zum Bereitstellen einer Schätzung von Atemsignalen von einer Person, wobei das Verfahren Folgendes umfasst:
- Empfangen von aufgezeichneten Geräuschen (110), die Geräusche umfassen, die von einem Brustkorb einer Person stammen und von biologischen Geweben der Person zu den Ohren der Person übertragen werden, wobei die aufgezeichneten Geräusche (1) vorher von mindestens einem Geräuschaufzeichnungselement aufgezeichnet werden, das im Inneren mindestens einer Ohrmuschel von Kopfhörern, die von der Person getragen werden, positioniert ist;
- Empfangen von Signalen von einem Beschleunigungsmesser und einem Gyroskop (3), die gleichzeitig mit den aufgezeichneten Geräuschen aufgezeichnet wurden;
- Extrahieren von Herzspitzen (130), die systolischen und diastolischen Geräuschen entsprechen, und Restgeräuschen (20), die durch die Atmung der Person erzeugte Informationen umfassen, aus den aufgezeichneten Geräuschen;
- Erfassen von Herzschlägen aus den Herzspitzen (30) und Berechnen von Intervallen zwischen den Herzschlägen aus den Herzschlägen;
- Extrahieren einer ersten Schätzung eines Atemsignals (41) aus den Intervallen (150) zwischen den Herzschlägen, die respiratorische Sinusarrhythmie (RSA) darstellen;
- Extrahieren einer zweiten Schätzung des Atemsignals (42) aus den Restgeräuschen (160);
- Extrahieren einer dritten Schätzung des Atemsignals (43) und von Bewegungsartefakten aus den Signalen des Beschleunigungsmessers und des Gyroskops (170);
- Berechnen (180) einer vierten Schätzung des Atemsignals (40) durch Kombinieren der ersten (41), der zweiten (42) und der dritten (43) Schätzung des Atemsignals, und
- Bereitstellen der vierten Schätzung des Atemsignals (40) zur Gesundheitsüberwachung.

2. Verfahren nach Anspruch 1, wobei das Extrahieren der Geräusche (130) der Herzspitzen einen Schritt des Verstärkens der Spitzen in den aufgezeichneten Geräuschen und einen Schritt des Erfassens der Geräusche der Herzspitzen unter Verwendung einer diskreten Wavelet-Transformation umfasst.

3. Verfahren nach einem der Ansprüche 1 und 2, wobei der Schritt des Extrahierens der ersten Schätzung des Atemsignals (150) die Anwendung einer Fast-FourierTransformation auf die Intervalle zwischen den Herzschlägen und die Auswahl der niederfrequenten Komponenten umfasst.

4. Verfahren nach einem der Ansprüche 1 bis 3, wobei der Schritt des Extrahierens der zweiten Schätzung des Atemsignals (160) aus den Restgeräuschen (20) unter Verwendung einer Zeitfrequenzanalyse und Periodizitätserfassung durchgeführt wird.

5. Verfahren nach einem der Ansprüche 1 bis 4, wobei der Schritt des Extrahierens der dritten Schätzung des Atemsignals (170) die Verwendung von Hauptkomponentenanalysezerlegung, schneller Fourier-Spektralberechnung und Komponentendetektion der Signale des Beschleunigungsmessers und des Gyroskops umfasst.

6. Verfahren nach einem der Ansprüche 1 bis 5, wobei ein Fusionsalgorithmus (182) verwendet wird, um die erste (41), die zweite (42) und die dritte Schätzung des Atemsignals (43) zu kombinieren.

7. Verfahren nach einem der Ansprüche 1 bis 6, ferner umfassend den Schritt des Empfangens von elektro-enzephalographischen Signalen von der Person, die gleichzeitig mit den aufgezeichneten Geräuschen aufgezeichnet werden.

8. Computerprogrammprodukt zum Überwachen von physiologischen Signalen von einer Person, wobei das Computerprogrammprodukt Anweisungen umfasst, die, wenn das Programm von einem Computer ausgeführt wird, den Computer dazu veranlassen, die Schritte des Verfahrens nach einem der Ansprüche 1 bis 7 durchzuführen.

9. System (S) zum Bereitstellen einer Schätzung von Atemsignalen von einer Person aus aufgezeichneten Geräuschen, umfassend:
- ein Eingabemodul (AM), das konfiguriert ist zum Empfangen von:
• aufgezeichneten Geräuschen (1), die unter Verwendung mindestens eines Geräuschaufzeichnungselements (REi) erlangt werden, das im Inneren mindestens einer Ohrmuschel von Kopfhörern, die von der Person getragen werden, positioniert ist, wobei das aufgezeichnete Geräusch von einem Brustkorb einer Person stammt und von biologischen Geweben der Person zu den Ohren der Person übertragen werden;
• Signalen von einem Beschleunigungsmesser und einem Gyroskop (3), wobei die Signale gleichzeitig mit den aufgezeichneten Geräuschen erlangt wurden;
- ein Extraktionsmodul (EM), das konfiguriert ist, um Herzspitzen (30), die systolischen und diastolischen Geräuschen entsprechen, und Restgeräusche (20), die durch die Atmung der Person erzeugte Informationen umfassen, aus den aufgezeichneten Geräuschen (1) zu extrahieren;
- ein Herzanalysemodul (CAM), das konfiguriert ist, um die Herzschläge aus den Herzspitzen (30) zu erfassen und Intervalle zwischen den Herzschlägen aus den Herzschlägen zu berechnen;
- ein Atmungsanalysemodul (RAM), das konfiguriert ist, um eine erste Schätzung eines Atemsignals von den Intervallen zwischen den Herzschlägen aus den aufgezeichneten Geräuschen (10) zu extrahieren, eine zweite Schätzung des Atemsignals aus Restgeräuschen (20) zu extrahieren, eine dritte Schätzung des Atemsignals und von Bewegungsartefakten aus den Signalen des Beschleunigungsmessers und des Gyroskops zu extrahieren und eine vierte Schätzung des Atemsignals, die die erste, die zweite und die dritte Schätzung des Atemsignals kombiniert, zu berechnen.

10. System nach Anspruch 9, ferner umfassend Kopfhörer (H), die zwei Ohrmuscheln (E) umfassen, die konfiguriert sind, um das Geräusch zu verstärken, das vom Brustkorb der Person stammt und von biologischen Geweben der Person zu den Ohren der Person übertragen wird.

11. System nach Anspruch 10, wobei das mindestens eine Geräuschaufzeichnungselement (REi) im Inneren mindestens einer der Ohrmuscheln (E) positioniert ist.

12. System nach einem der Ansprüche 10 und 11, ferner umfassend ein externes Geräuschaufzeichnungselement (REe), das außerhalb mindestens einer der Ohrmuscheln (E) positioniert ist, so dass Geräusche aufgezeichnet werden, die sich in einer Umgebung außerhalb der mindestens einen Ohrmuschel (2) ausbreiten.

13. System nach einem der Ansprüche 10 bis 12, wobei die Ohrmuscheln (E) der Kopfhörer (H) kopfumschließende Kopfhörer oder ohraufliegende Kopfhörer sind.

14. System nach einem der Ansprüche 12 und 13, ferner umfassend ein Rauschunterdrückungsmodul (DM), das konfiguriert ist, um die Geräusche zu empfangen, die sich in der Umgebung außerhalb der Ohrmuscheln (2) ausbreiten, und unter Verwendung der Geräusche, die sich in der Umgebung außerhalb der Ohrmuscheln (2) ausbreiten, einen Teil eines Rauschens aus den aufgezeichneten Geräuschen (1) zu entfernen.

## Revendications

1. Procédé mis en œuvre par ordinateur pour fournir une estimation de signaux respiratoires d'un sujet, ledit procédé comprenant :
- recevoir des sons enregistrés (110) comprenant des sons provenant d'une poitrine d'un sujet et transmis par des tissus biologiques du sujet aux oreilles du sujet, dans lequel lesdits sons enregistrés (1) sont préalablement enregistrés par au moins un élément d'enregistrement sonore positionné à l'intérieur d'au moins un écouteur d'un casque porté par le sujet ;
- recevoir des signaux provenant d'un accéléromètre et d'un gyroscope (3) qui ont été enregistrés simultanément avec les sons enregistrés ;
- extraire des sons enregistrés des pics cardiaques (130), correspondant à des sons systoliques et diastoliques, et des sons résiduels (20) comprenant des informations générées par respiration du sujet ;
- détecter des battements cardiaques à partir des pics cardiaques (30) et calculer des intervalles inter-battements à partir des battements cardiaques ;
- extraire une première estimation d'un signal respiratoire (41) à partir des intervalles inter-battements (150) présentant une arythmie sinusale respiratoire (RSA) ;
- extraire une deuxième estimation du signal respiratoire (42) à partir de sons résiduels (160) ;
- extraire une troisième estimation du signal respiratoire (43) et des artefacts de mouvement à partir des signaux de l'accéléromètre et du gyroscope (170) ;
- calculer (180) une quatrième estimation du signal respiratoire (40) en combinant la première (41), la deuxième (42) et la troisième (43) estimation du signal respiratoire, et
- fournir la quatrième estimation du signal respiratoire (40) pour une surveillance de santé.

2. Procédé selon la revendication **1**, dans lequel l'extraction des sons de pics cardiaques (130) comprend une étape d'augmentation des pics dans les sons enregistrés et une étape de détection des sons de pics cardiaques utilisant une transformée en ondelettes discrètes.

3. Procédé selon l'une ou l'autre des revendications **1** ou **2**, dans lequel l'étape d'extraction de la première estimation du signal respiratoire (150) comprend l'application d'une transformation de Fourier rapide aux intervalles inter-battements rééchantillonnés et la sélection de la composante basse fréquence.

4. Procédé selon l'une quelconque des revendications **1** à **3**, dans lequel l'étape d'extraction de la deuxième estimation du signal respiratoire (160) à partir des sons résiduels (20) est réalisée en utilisant une analyse temps-fréquence et une détection de périodicité.

5. Procédé selon l'une quelconque des revendications **1** à **4**, dans lequel l'étape d'extraction de la troisième estimation du signal respiratoire (170) comprend l'utilisation d'une décomposition par analyse en composantes principales, d'un calcul spectral de Fourier rapide et d'une détection de composantes des signaux de l'accéléromètre et du gyroscope.

6. Procédé selon l'une quelconque des revendications **1** à **5**, dans lequel un algorithme de fusion (182) est utilisé pour combiner la première (41), la deuxième (42) et la troisième estimation du signal respiratoire (43).

7. Procédé selon l'une quelconque des revendications **1** à **6**, comprenant en outre l'étape de réception de signaux électroencéphalographiques du sujet enregistrés simultanément aux sons enregistrés.

8. Produit de programme informatique pour surveiller des signaux physiologiques d'un sujet, le produit de programme informatique comprenant des instructions qui, lorsque le programme est exécuté par un ordinateur, amènent l'ordinateur à réaliser les étapes du procédé selon l'une quelconque des revendications **1** à **7.**

9. Système (S) destiné à fournir une estimation de signaux respiratoires d'un sujet à partir de sons enregistrés comprenant :
- un module d'entrée (AM) configuré pour recevoir :
• des sons enregistrés (1) acquis en utilisant au moins un élément d'enregistrement sonore (REi) positionné à l'intérieur d'au moins un écouteur d'un casque porté par le sujet, ledit son enregistré (1) provenant d'une poitrine du sujet et étant transmis par des tissus biologiques du sujet aux oreilles du sujet ;
• des signaux provenant d'un accéléromètre et d'un gyroscope (3), lesdits signaux ayant été acquis simultanément avec les sons enregistrés ;
- un module d'extraction (EM) configuré pour extraire des sons enregistrés (1) des pics cardiaques (30), correspondant à des sons systoliques et diastoliques, et des sons résiduels (20) comprenant des informations générées par la respiration du sujet ;
- un module d'analyse cardiaque (CAM) configuré pour détecter les battements cardiaques à partir des sons de pics cardiaques (30) et calculer les intervalles inter-battements à partir des battements cardiaques ;
- un module d'analyse respiratoire (RAM) configuré pour extraire des sons enregistrés (10) une première estimation d'un signal respiratoire à partir des intervalles inter-battements, extraire une deuxième estimation du signal respiratoire à partir de sons résiduels (20), extraire une troisième estimation du signal respiratoire et des artefacts de mouvement à partir des signaux de l'accéléromètre et du gyroscope, et calculer une quatrième estimation du signal respiratoire combinant la première, la deuxième et la troisième estimation du signal respiratoire.

10. Système selon la revendication **9**, comprenant en outre un casque (H) comprenant deux oreillettes (E) configurées pour amplifier le son provenant de la poitrine du sujet et étant transmis par les tissus biologiques du sujet aux oreilles du sujet.

11. Système selon la revendication **10**, dans lequel le au moins un élément d'enregistrement sonore (REi) est positionné à l'intérieur d'au moins un des écouteurs (E).

12. Système selon l'une ou l'autre des revendications **10** ou **11**, comprenant en outre un élément d'enregistrement sonore externe (REe) positionné à l'extérieur d'au moins l'une des oreillettes (E) de manière à enregistrer les sons se propageant dans un environnement externe à la au moins une oreillette (2).

13. Système selon l'une quelconque des revendications **10** à **12**, dans lequel les oreillettes (E) du casque (H) sont un casque circum-aural ou un casque supra-aural.

14. Système selon l'une ou l'autre des revendications **12** ou **13**, comprenant en outre un module de débruitage (DM) configuré pour recevoir les sons se propageant dans l'environnement extérieur aux oreillettes (2) et retirer des sons enregistrés (1) une partie d'un bruit utilisant lesdits sons se propageant dans l'environnement extérieur aux oreillettes (2).
